(19) Europäisches Patentamt

European Patent Office

Office européen des brevets

(11) **EP 1 305 994 B1**

(12) **EUROPÄISCHE PATENTSCHRIFT**

(45) Veröffentlichungstag und Bekanntmachung des Hinweises auf die Patenterteilung:
**11.05.2005 Patentblatt 2005/19**

(51) Int Cl.$^7$: **A01D 41/127**

(21) Anmeldenummer: **02102471.6**

(22) Anmeldetag: **22.10.2002**

(54) **Kornfeuchtigkeitssensor**

Grain Moisture Sensor

Capteur d'humidité de grain

(84) Benannte Vertragsstaaten:
**BE DE DK FR IT**

(30) Priorität: **25.10.2001 US 3884**

(43) Veröffentlichungstag der Anmeldung:
**02.05.2003 Patentblatt 2003/18**

(60) Teilanmeldung:
**04100529.9 / 1 421 841**

(73) Patentinhaber: **DEERE & COMPANY**
**Moline, Illinois 61265-8098 (US)**

(72) Erfinder:
• **Rains, Gerald E.**
  **Bettendorf , IA 52722 (US)**
• **Phelan, James Joseph**
  **Bettendorf , IA 52722 (US)**
• **Slavens, Zachary W.**
  **Bettendorf , IA 52722 (US)**
• **Kozicki, Andrzej**
  **Milan , IL 61264 (US)**
• **Funk, Robert C.**
  **Auburn , IL 62615 (US)**

(74) Vertreter: **Holst, Sönke, Dr. et al**
**Deere & Company,**
**European Office,**
**Patent Department**
**Steubenstrasse 36-42**
**68163 Mannheim (DE)**

(56) Entgegenhaltungen:
**EP-A- 0 389 320     EP-A- 1 072 885**
**CA-A- 2 182 989     US-A- 5 092 819**
**US-A- 5 957 773**

**Beschreibung**

Die Erfindung betrifft einen Kornfeuchtigkeitssensor für einen Mähdrescher, mit einer elektrisch beaufschlagten Platte, einer Sensorplatte, die in der Nähe der elektrisch beaufschlagten Platte angeordnet und zu ihr im Wesentlichen parallel orientiert ist, um im Zwischenraum zwischen den Platten die Kapazität zu messen, einer Füllstandssensorplatte, die in der Nähe der Sensorplatte und im Wesentlichen parallel zur elektrisch beaufschlagten Platte angeordnet ist, um zu erfassen, ob der Zwischenraum mit Korn gefüllt ist und mit einer Steuerung des Kornfeuchtigkeitssensors.

Kornfeuchtigkeitssensoren werden in Mähdreschern verwendet, insbesondere in Präzisionslandwirtschaftsanwendungen. Kontinuierliche oder momentane Kornfeuchtigkeitsmessungen erlauben einem Bediener, die Feuchtigkeit des Korns während des Erntens zu beobachten. In Verbindung mit einer Einrichtung zur Ortsbestimmung, z. B. GPS, kann ein Feuchtigkeitssensor zur Feuchtigkeitskartierung verwendet werden. Außerdem werden Feuchtigkeitssensoren in Ertragskartierungsanwendungen genutzt. Wenn sie in Kombination mit einem Kornflusssensor benutzt werden, wird die Information des Feuchtigkeitssensors häufig benutzt, die Menge trockenen Getreidevolumens auf einem Feld und das Getreidevolumen auf einer Flächeneinheit basierend auf der Menge feuchten Getreides und des Feuchtigkeitsgehaltes zu berechnen.

In der EP 0 389 320 A wird ein Gerät eingangs genannter Art zur Erfassung der Feuchtigkeit eines kornförmigen Produkts beschrieben. Das Gerät weist zwei mit einer Steuerung verbundene Kondensatorenplattenpaare auf, zwischen denen das Produkt eingeführt wird. Das untere Kondensatorenplattenpaar dient zur Messung der Feuchtigkeit des Produkts, während das obere Kondensatorenplattenpaar zur Überprüfung dient, ob die Zelle mit Produkt gefüllt ist. Dabei wird nur geprüft, ob die Kapazität des oberen Kondensatorenplattenpaars von einem leeren Kondensatorenplattenpaar abweicht oder nicht. Bei einer derartigen, groben Überprüfung sind Fehler zu erwarten, die beispielsweise durch den Leitwert von an den Wänden der Zelle haftenden Verunreinigungen verursacht werden können, was dazu führt, dass eine tatsächlich nicht gefüllte Zelle aufgrund der durch die Verunreinigungen bedingten Ströme als gefüllt angesehen wird.

Die der Erfindung zu Grunde liegende Aufgabe wird darin gesehen, einen verbesserten Kornfeuchtigkeitssensor für einen Mähdrescher für die oben genannten Anwendungen bereitzustellen.

Diese Aufgabe wird erfindungsgemäß durch die Lehre des Patentanspruchs 1 gelöst, wobei in den weiteren Patentansprüchen Merkmale aufgeführt sind, die die Lösung in vorteilhafter Weise weiterentwickeln.

Der erfindungsgemäße Kornfeuchtigkeitssensor umfasst eine Zelle zur Aufnahme von Korn, die eine elektrisch beaufschlagte Platte umfasst, an die Anregungsspannungen angelegt werden, eine Sensorplatte in der Nähe zur elektrisch beaufschlagten Platte, die sich parallel dazu erstreckt, um den durch die Zelle fließenden Strom zu messen und eine Füllstandssensorplatte nahe der Sensorplatte, um festzustellen, wenn die Zelle gefüllt ist. Die Füllstandssensorplatte befindet sich in der Regel an der Einlassseite der Zelle. Der sie durchfließende Strom gibt einen Hinweis auf den Füllstand der Zelle: wenn die auf die Fläche bezogene Stromdichte an der Füllstandssensorplatte der Stromdichte an der Sensorplatte entspricht, ist die Zelle gefüllt.

Auf diese Weise kann eine Steuerung des Kornfeuchtigkeitssensors erkennen, dass eine Feuchtigkeitsmessung durchführbar ist. Fehlmessungen durch eine nur teilweise gefüllte Zelle werden vermieden. Eventuelle korndurchlässige Lecks in der Zelle können leicht erkannt werden.

Vorzugsweise ist der Sensorplatte und auch der Füllstandssensorplatte eine Abschirmung zugeordnet. Die Sensorplatte ist zwischen der Abschirmung und der elektrisch beaufschlagten Platte angeordnet. Die Abschirmung ist elektrisch von der Sensorplatte isoliert, befindet sich aber auf demselben Potential. Die Abschirmung ist parallel zur Sensorplatte orientiert und von größeren Abmessungen als die Sensorplatte, erstreckt sich somit über ihre Ränder hinaus, um das elektrische Feld zu formen. Das Vorhandensein der Abschirmung stellt den Vorteil gerader elektrischer Feldlinien bereit, die senkrecht zur Sensorplatte verlaufen und über das gesamte Volumen zwischen den parallelen Platten eine gleichförmige Dichte aufweisen. Das hat verminderte Randeffekte und eine gleichförmige elektrische Felddichte zur vorteilhaften Folge, so dass über die gesamte Zelle eine konstante Empfindlichkeit erreicht wird. Außerdem schirmt die Abschirmung die Sensorplatte von externen elektrischen Feldern ab, die von anderen Feldern als der elektrisch beaufschlagten Platte erzeugt werden.

Zweckmäßigerweise ist ein Temperatursensor vorgesehen, der die Temperatur des Korns in der Zelle zumindest näherungsweise erfasst. Sein Messwert wird bei der Massenberechnung herangezogen.

Die vorliegende Erfindung ermöglicht eine Messung der komplexen Dielektrizitätskonstante des Korns. Es wird außerdem ermöglicht, diese bei unterschiedlichen Frequenzen zu messen. Dadurch ergibt sich der Vorteil, Variationen in der Korndichte und Leitfähigkeitseffekte auszugleichen, was bei mobilen Feuchtigkeitsfühlern besonders wichtig ist, wie bei der Verwendung eines Feuchtigkeitsfühlers an einem Mähdrescher. Es wird vorgeschlagen, dass ein Schaltkreis sowohl die Real- als auch die Imaginärteile sowohl der Anregungsspannung als auch des Stroms in der Sensorplatte misst. Aus diesen Werten wird die komplexe Dielektrizitätskonstante der Zelle (oder ein damit im Zusammenhang stehender Wert) ermittelt. Die Messungen können für die leere und die mit Korn gefüllte Zelle wiederholt werden. Insbesondere, wenn eine leere Zelle nicht verfügbar ist, werden anstelle dessen kalibrierte Referenzleitwerte verwendet. Aus diesen Messungen kann die komplexe Dielektrizitätskonstante des Korns errechnet werden. Zur Messung der Real- und Imaginärteile der Spannung und des Stroms können Mischer verwendet werden. Dieses synchrone Detektionsverfahren, wie es auch an Lock-in-Verstärkern benutzt wird, hat einen sehr schmalbandigen Filtereffekt, der den Störgeräuscheinfluss auf die Messung wesentlich reduziert. Zur Messung des Stroms an der Sensorplatte wird ein Verfahren zur Messung niedriger Ströme mit virtuellem Erdpotential genutzt, um die Vorteile einer wesentlichen Verminderung des Einflusses parasitärer Elemente am Strommessknoten zu erzielen.

Außerdem können die Messungen mit kalibrierten Referenzen korrigiert werden, um alle Änderungen der Umgebungsbedingungen ausgleichen zu können, die die Eigenschaften des Schaltkreises beeinflussen. Das stellt den Vorteil einer Absicherung stabiler und wiederholbarer Ergebnisse bereit. Wenn verschiedene Referenzen benutzt werden, können eine oder mehrere Verzerrungscharakteristika der Messung der Real- und Imaginärteile erfasst und bei der Auswertung berücksichtigt werden. Als Referenzleitwerte können die leere Zelle, Kondensatoren und/oder Induktivitäten oder beliebige Kombinationen daraus verwendet werden.

Der Kornfeuchtigkeitssensor kann grundsätzlich an beliebigen Stellen des Mähdreschers angebracht werden. Vorzugsweise wird er jedoch innerhalb des Korntanks, beispielsweise versetzt zur Vorderseite des Übergangsgehäuses des Körnerelevators angeordnet. Ein Vorteil ist, dass ein Zugang zum Kornfeuchtigkeitssensor möglich ist, wenn erforderlich, und dass sich alle eventuellen Lecks der Messzelle im Korntank befinden, so dass kein Korn verloren gehen kann. Ein weiterer Vorteil ist, dass die Zelle des Kornfeuchtigkeitssensors aktiv (durch den Körnerelevator) mit Korn gefüllt wird. An dieser Stelle kann die Zelle unabhängig von (Hang-) Neigungsbedingungen des Mähdreschers gefüllt werden. Es kann ein (z. B. elektrisch, pneumatisch oder hydraulisch) fremdkraftbetriebener Aktor vorgesehen sein, der zwischen einer ersten und einer zweiten Position bewegbar ist. In der ersten Position ermöglicht er das Füllen der Zelle mit Korn (bzw. füllt sie aktiv) und in der zweiten Position entleert er die Zelle.

Bei der Anbringung der Zelle im Korntank, der Abschirmung, der Messung der komplexen Leitwerte mittels eines Referenzleitwerts und der Messung der Ströme und Spannungen mit Synchrondetektoren handelt es sich um Merkmale, denen selbstständiger erfinderischer Rang zukommt.

In den Zeichnungen ist ein nachfolgend näher beschriebenes Ausführungsbeispiel der Erfindung dargestellt. Es zeigt:

Fig. 1A    eine Seitenansicht eines Mähdreschers mit einem erfindungsgemäßen Kornfeuchtigkeitssensor,

Fig. 1B    eine Seitenansicht eines erfindungsgemäßen Kornfeuchtigkeitssensors, der an einem Mähdrescher angebracht und in einer Füllposition ist,

Fig. 1C    eine Seitenansicht eines erfindungsgemäßen Kornfeuchtigkeitssensors, der an einem Mähdrescher angebracht und in einer Messposition ist,

Fig. 2    ein seitlicher Querschnitt durch die Zelle eines erfindungsgemäßen Kornfeuchtigkeitssensors,

Fig. 3    ein seitlicher Querschnitt durch die Zelle aus Figur 2, in der die Äquipotentiallinien des elektrischen Feldes dargestellt sind, das entsteht, wenn eine Anregungsspannung an die elektrisch beaufschlagte Platte angelegt wird,

Fig. 4    ein schematisches Schaltdiagramm eines Modells der erfindungsgemäßen Zelle,

Fig. 5    ein Blockdiagramm des Admittanzmeßkreises eines erfindungsgemäßen Kornfeuchtesensors, und

Fig. 6A und 6B    Blockdiagramme eines erfindungsgemäßen Feuchtesensorschaltkreises.

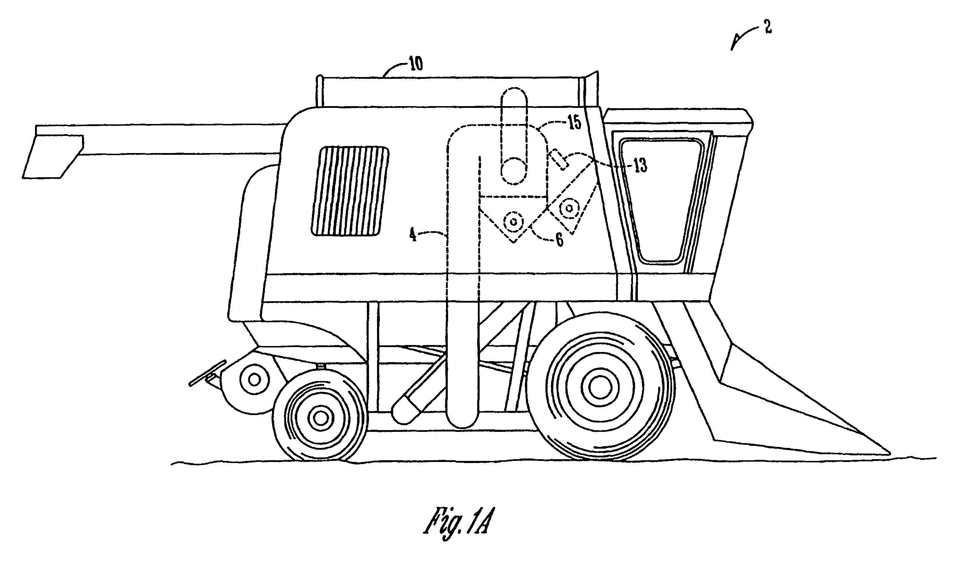

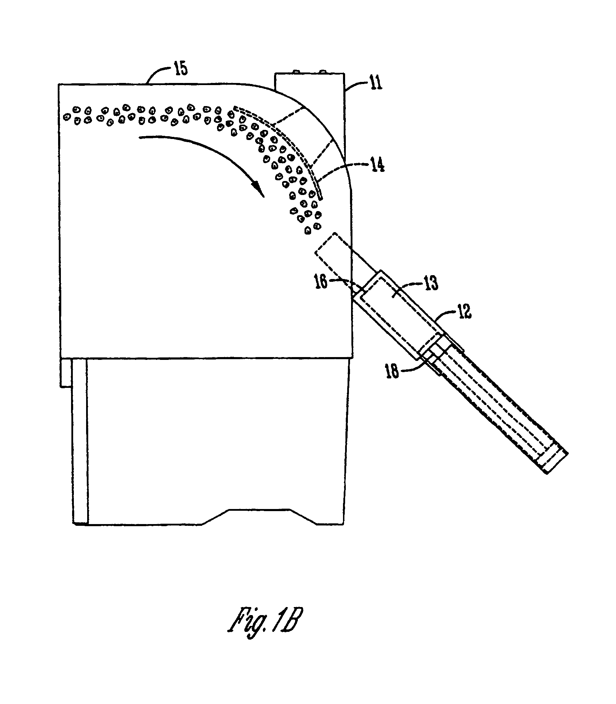

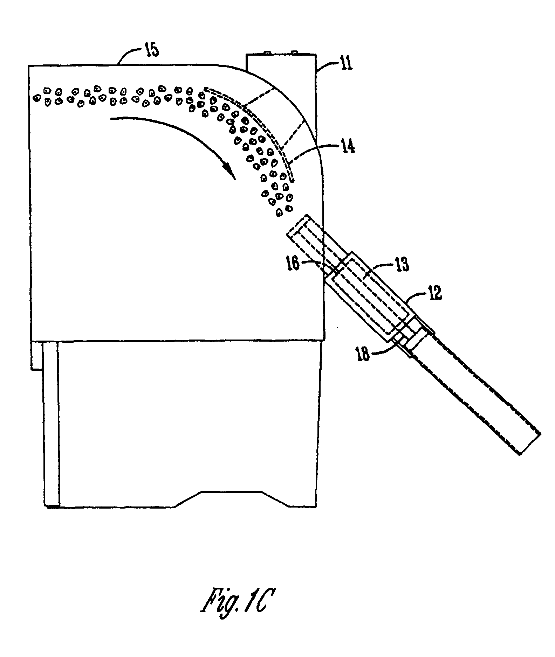

Die Figur 1A zeigt einen Mähdrescher mit einem erfindungsgemäßen Kornfeuchtigkeitssensor. In der Figur 1A ist der Mähdrescher 2 mit einem Korntank 10 dargestellt. Außerdem wird ein Elevator 4 für sauberes Korn gezeigt. Korn aus dem Elevator 4 für sauberes Korn bewegt sich zum Übergangsgehäuse 15 des Korntanks 10. Der Sumpf 6 des Übergangsgehäuses 15 wird auch gezeigt.

Die Figuren 1B und 1C zeigen Seitenansichten eines erfindungsgemäßen Kornfeuchtigkeitssensors, der an einem Mähdrescher angebracht ist. Der dargestellte Korntank 10 umfasst den Kornfeuchtigkeitssensor 12. Der Kornfeuchtigkeitssensor 12 ist im Korntank 10 des Mähdreschers 2 nahe dem Massenflusssensor 11 angeordnet. Die Öffnung 16 der Zelle 13 ist unterhalb der Prallplatte 14 im Übergangsgehäuse 15 angeordnet. Obwohl eine Prallplatte 14 dargestellt ist, beinhaltet die vorliegende Erfindung, dass andere Deflektoren verwendet werden können. An dieser Stelle wird die Zelle 13 aufgrund der direkten oder indirekten Geschwindigkeiten des Korns aktiv gefüllt, die durch die (nicht gezeigten) Paddel des Elevators 4 für sauberes Korn erzeugt werden. Dies erlaubt, dass die Zelle 13 mit hohen Raten gefüllt wird. Dies vermindert jegliche mit langsamen Zykluszeiten verbundene Probleme, die mit Bedingungen kleiner Flüsse verbunden sind, da hier die Zelle 13 wegen ihrer Anordnung innerhalb des vom Elevator 4 für sauberes Korn erzeugten Stroms mit einer hohen Rate gefüllt wird. Die Zelle 13 ist mit ihrem Einlass ausgerichtet. Ein Stößel mit Kolben 18 mit einem elektrischen Aktor wird verwendet, die Kornprobe zurück aus der Einlassöffnung 16 zu zwingen. In der Figur 1C sind die Zelle 13 und der Stößel mit Kolben 18 in einer Messposition.

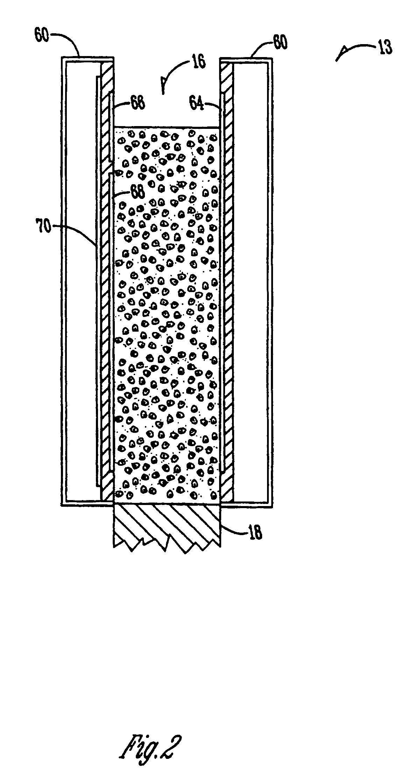

Die Figur 2 stellt eine Seitenansicht einer erfindungsgemäßen Zelle 13 dar. Die Öffnung 16 der Zelle 13 oder der Zwischenraum ist mit Korn gefüllt. An beiden Seiten des Zwischenraums sind parallele Kondensatorplatten 64 und 68. Die elektrisch beaufschlagte Platte 64 ist die Platte, an die eine Anregungsspannung angelegt ist. Die Sensorplatte 68 ist die Platte, an der der Strom gemessen wird, der die Zelle 13 durchströmt. Die Füllstandssensorplatte 66 ist nahe der Sensorplatte 68 und parallel zur elektrisch beaufschlagten Platte 64.

Die Füllstandssensorplatte 66 zur Erfassung des Füllstands der Zelle 13 ist von einem Viertel der Größe der Sensorplatte 68. Um festzustellen, ob oder wann die Zelle 13 gefüllt ist, sollte die Füllstandssensorplatte 66 einen Messwert bereitstellen, der ein Viertel des Messwerts der Sensorplatte 68 ist. Obwohl in dieser Ausführungsform die Füllstandssensorplatte 66 ein Viertel der Größe der Sensorplatte 68 hat, beinhaltet die vorliegende Erfindung verschiedene Änderungen in den Größen der Platten 64, 66 und 68. Dies ist nur ein Beispiel einer geeigneten und nützlichen relativen Größe.

Die Abschirmung 70 ist strategisch günstig hinter der Sensorplatte 68 und der Füllstandssensorplatte 66 angeordnet. Die Abschirmung 70 ist parallel zur Sensorplatte 68 und größer dimensioniert, um das elektrische Feld zu formen. Außerdem schirmt die Abschirmung 70 die Sensorplatte 68 von äußeren elektrischen Feldern ab, die von anderen Quellen als der elektrisch beaufschlagten Platte 64 erzeugt werden.

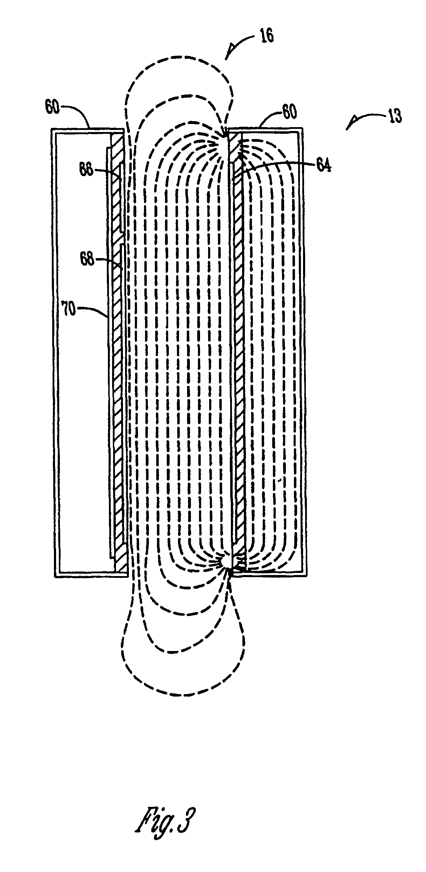

Die Figur 3 illustriert die Zelle 13 des Feuchtigkeitssensors 12 mit den Äquipotentiallinien des elektrischen Felds, das erzeugt wird, wenn ein Anregungssignal an die elektrisch beaufschlagte Platte 64 angelegt wird. Wegen der räumlichen Anordnung der Abschirmung 70, die von der Sensorplatte 68 elektrisch isoliert ist, aber auf demselben Potential liegt, ist die Wirkung auf die elektrischen Feldlinien in der Nachbarschaft der Sensorplatte 68, dass ein Äquivalent eines idealen Parallelplattenkondensators ohne Randeffekte erzeugt wird. Die elektrischen Feldlinien sind gerader Natur und senkrecht zur Sensorplatte 68 und zur Füllstandssensorplatte 66. Außerdem sind die elektrischen Feldlinien in der gesamten Region zwischen den parallelen Platten 66, 68 gleichförmiger Dichte. Das Ergebnis ist, dass Randeffekte vermindert sind. Randeffekte erzeugen unkontrollierbare Einflüsse auf die Messungen durch von Korn sich unterscheidende Materialien, die sich nahe aber außerhalb der Zelle 13 befinden. Hier zeigen die geraden elektrischen Feldlinien innerhalb der Zelle 13, dass die Zelle 13 im Wesentlichen gegen derartige Einflüsse immun ist. Außerdem stellt die gleichförmige elektrische Felddichte eine konstante Empfindlichkeit in der gesamten Zelle 13 bereit. Zusätzlich sind die ganze Zelle 13 und die Elektronik in einem Metallgehäuse 60 enthalten. Das Metallgehäuse 60 dient als Schirm gegen elektromagnetische Störungen und isoliert weiterhin die ganze Zelle 13 von anderen Quellen elektromagnetischer Energie.

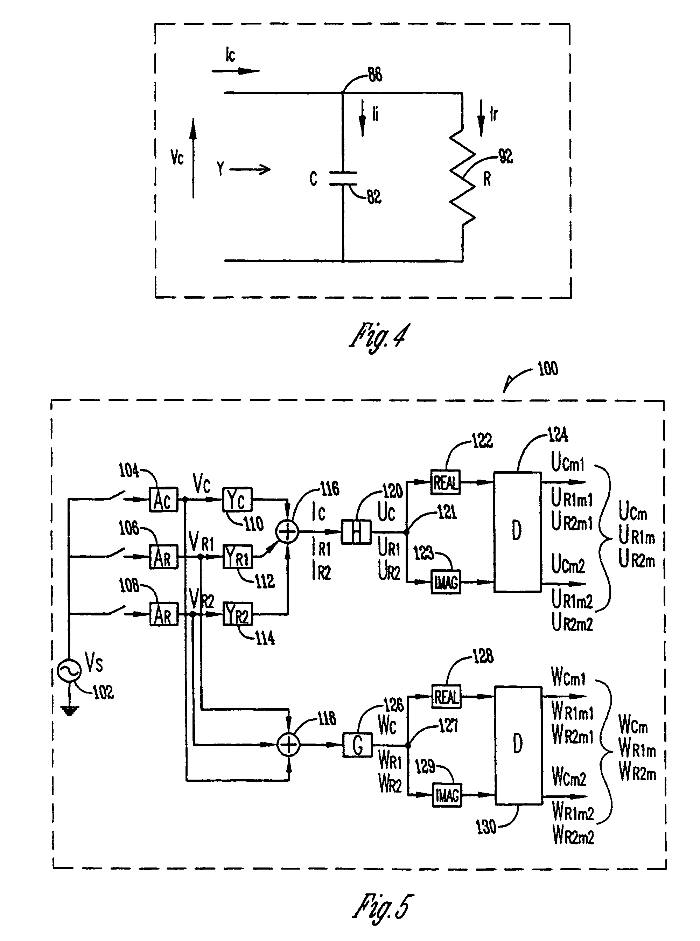

Die vorliegende Erfindung ermöglicht Kornfeuchtigkeitsmessungen basierend auf der Messung der komplexen relativen Dielektrizitätskonstante des Korns, die im Folgenden als komplexe Dielektrizitätskonstante bezeichnet wird. Die Figur 4 illustriert ein schematisches Diagramm eines Schaltkreises, der der erfindungsgemäßen Kondensatorzelle 13 elektrisch äquivalent ist. Diese Ersatzschaltung umfasst einen idealen Kondensator 82 mit einem Wert C, der parallel mit einem idealen Widerstand 92 mit dem Wert R geschaltet ist. Der ideale Kondensator 82 repräsentiert die kapazitive oder energiespeichernde Eigenschaft der Zelle 13 und der ideale Widerstand 92 repräsentiert die konduktive oder energiedissipative Eigenschaft der Zelle 13. C und R hängen von der Anregungsfrequenz und der Feuchtigkeit, Temperatur und bestimmten anderen Eigenschaften des Korns ab.

Der komplexe Leitwert der Zelle 13 ist

$$Y = (1/R) + j\omega C,$$

wobei $\omega = 2\pi f$, f die Anregungsfrequenz und j die imaginäre Einheit ist. Wenn die Zelle 13 leer ist, hat sie im Wesentlichen keine energiedissipativen Eigenschaften. Ihr Leitwert ist sehr nahe dem eines idealen Kondensators mit dem Wert $C_{CE}$:

$$Y_{CE} = j\omega C_{CE}.$$

Wenn die Zelle 13 mit Korn gefüllt ist, hat sie sowohl energiedissipative und energiespeichernde Eigenschaften. Ihr Leitwert ist

$$Y_{CF} = (1/R_{CF}) + j\omega C_{CF}.$$

Durch Teilen des Leitwerts der gefüllten Zelle 13 durch den der leeren Zelle 13 erhält man:

$$Y_{CF}/Y_{CE} = C_{CF}/C_{CE} - j/\omega C_{CE} R_{CE} == \varepsilon$$

Dieses Verhältnis ist die komplexe Dielektrizitätskonstante des Korns. Die komplexe Dielektrizitätskonstante ist eine intrinsische Materialeigenschaft und hängt nur von der Anregungsfrequenz und der Feuchtigkeit, Temperatur und bestimmter anderer Eigenschaften des Korns ab. Sie ist unabhängig von den Abmessungen und der Form der Zelle 13. Die komplexe Dielektrizitätskonstante wird im Allgemeinen folgendermaßen geschrieben:

$$\varepsilon = \varepsilon' - j\,\varepsilon''.$$

wobei $\varepsilon'$ die Dielektrizitätskonstante und $\varepsilon''$ der Verlustfaktor ist.

Es ist eine Aufgabe des Schaltkreises der vorliegenden Erfindung, den Leitwert der leeren Zelle 13 und der vollen Zelle 13 zu messen, um die obigen Gleichungen zu verwenden, um die komplexe Dielektrizitätskonstante des Korns zu berechnen. Wie in der Figur 4 gezeigt, wird die komplexe Anregungsspannung $V_C$ über den Schaltkreis gelegt. Der entstehende komplexe Strom $I_C$ fließt durch den Schaltkreis. $V_C$ hat eine reelle Komponente $V_r$ und eine imaginäre Komponente $V_i$:

$$V_C = V_r + j\,V_i$$

$I_C$ hat eine reelle Komponente $I_r$ und eine imaginäre Komponente $I_i$:

$$I_C = I_r + j\,I_i$$

Durch die Messung von $V_r$, $V_i$, $I_r$ und $I_i$ kann der komplexe Leitwert Y unter Verwendung komplexer Arithmetik berechnet werden:

$$Y = I_c/V_c = (I_r + jI_i)/(V_r + jV_i).$$

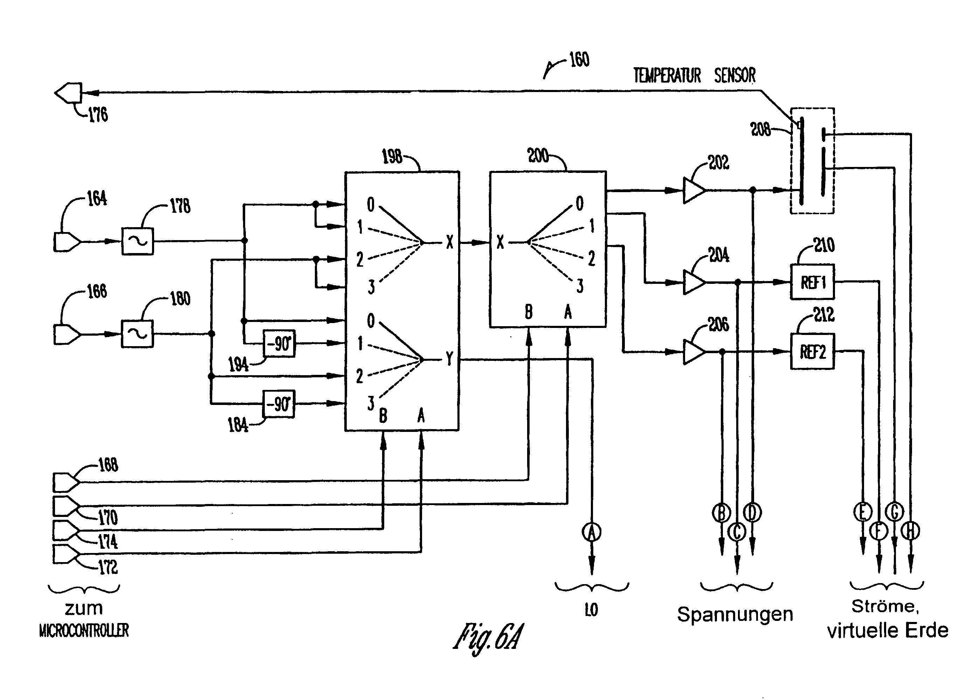

Die Figur 5 illustriert den im erfindungsgemäßen Kornfeuchtigkeitssensor verwendeten Schaltkreis zur Messung des Leitwerts. In der Figur 5 ist der Schaltkreis 100 zur Messung des Leitwerts gezeigt. Zu Erklärungszwecken werden die folgenden Definitionen verwendet:

$U_{R1m}$ wird als gemessener Wert des unten definierten $U_{R1}$ definiert.
$U_{R1m1}$ ist der Realteil (gleichphasiger Signalwert) von $U_{R1m}$.
$U_{R1m2}$ ist der Imaginärteil (Quadratur-Signalwert) von $U_{R1m}$.

$U_{R2m}$ wird als gemessener Wert des unten definierten $U_{R2}$ definiert.
$U_{R2m1}$ ist der Realteil (gleichphasiger Signalwert) von $U_{R2m}$.
$U_{R2m2}$ ist der Imaginärteil (Quadratur-Signalwert) von $U_{R2m}$.

$UC_m$ wird als gemessener Wert des unten definierten $U_C$ definiert.
$U_{Cm1}$ ist der Realteil (gleichphasiger Signalwert) von $U_{Cm}$.
$U_{Cm2}$ ist der Imaginärteil (Quadratur-Signalwert) von $U_{Cm}$.

$W_{R1m}$ wird als gemessener Wert des unten definierten $W_{R1}$ definiert.
$W_{R1m1}$ ist der Realteil (gleichphasiger Signalwert) von $W_{R1m}$.
$W_{R1m2}$ ist der Imaginärteil (Quadratur-Signalwert) von $W_{R1m}$.

$W_{R2m}$ wird als gemessener Wert des unten definierten $W_{R2}$ definiert.

$W_{R2m1}$ ist der Realteil (gleichphasiger Signalwert) von $W_{R2m}$.
$W_{R2m2}$ ist der Imaginärteil (Quadratur-Signalwert) von $W_{R2m}$.

$W_{Cm}$ wird als gemessener Wert des unten definierten $W_C$ definiert.
$W_{Cm1}$ ist der Realteil (gleichphasiger Signalwert) von $W_{Cm}$.
$W_{Cm2}$ ist der Imaginärteil (Quadratur-Signalwert) von $W_{Cm}$.

$U_C$ ist als komplexer Spannungswert definiert, der den die Zelle 13 durchfließenden Strom repräsentiert.
$U_{R1}$ ist als komplexer Spannungswert definiert, der den die erste Referenz durchfließenden Strom repräsentiert.
$U_{R2}$ ist als komplexer Spannungswert definiert, der den die zweite Referenz durchfließenden Strom repräsentiert.

$W_C$ ist als komplexer Spannungswert definiert, der die an der Zelle 13 anliegende Spannung repräsentiert.
$W_{R1}$ ist als komplexer Spannungswert definiert, der die an der ersten Referenz anliegende Spannung repräsentiert.
$W_{R2}$ ist als komplexer Spannungswert definiert, der die an der zweiten Referenz anliegende Spannung repräsentiert.

$I_C$ ist der komplexe, durch die Zelle fließende Strom.
$I_{R1}$ ist der komplexe, durch die erste Referenz fließende Strom.
$I_{R2}$ ist der komplexe, durch die zweite Referenz fließende Strom.

ist die komplexe Spannung an der Zelle.
$V_{R1}$ ist die komplexe Spannung an der ersten Referenz.
$V_{R2}$ ist die komplexe Spannung an der zweiten Referenz.

$Y_C$ ist der komplexe Leitwert der Zelle.

$Y_{R1}$ ist der komplexe Leitwert der ersten Referenz.
$Y_{R2}$ ist der komplexe Leitwert der zweiten Referenz.

$H$ ist die Übertragungsfunktion des Schaltkreises, der komplexe Strommessungen durchführt.
$G$ ist die Übertragungsfunktion des Schaltkreises, der komplexe Spannungsmessungen durchführt.
$V_S$ ist die erzeugte Quellenspannung.

$A_C$ ist die Übertragungsfunktion der Zellenbetriebsspannung.
$A_R$ ist die Übertragungsfunktion der Referenzbetriebsspannung.

D ist die Übertragungsfunktion der Phasen- und Gewinnfehlanpassung zwischen den gemessenen reellen (phasengleichen) und gemessenen imaginären (Quadratur-) Komponenten des komplexen Stroms und der Spannung. Diese Fehlanpassung wird durch Fehler in den Schaltkreiselementen bedingt, die die Messung durchführen. D ist auch als "Mischertransformationsmatrix" bekannt. Es ist Aufgabe der vorliegenden Erfindung, den Wert von D zu messen und seinen Einfluss auszugleichen.

Im Schaltkreis 100 zur Messung des Leitwerts wird eine erzeugte Quellspannung 102 ($V_S$) selektiv an die Zelle 13 oder an eine einer Mehrzahl von Referenzen über eine zugeordnete Übertragungsfunktion angelegt, wie durch die Bezugszeichen 104, 106 und 108 dargestellt. Wenn $V_S$ an die Übertragungsfunktion $A_C$ 104 angelegt wird, wird eine Spannung $V_C$ erzeugt, die an den komplexen Leitwert der Zelle $Y_C$ 110 angelegt wird. Wenn analog die Spannung 102 ($V_S$) an eine erste Übertragungsfunktion $A_R$ 106 angelegt wird, wird die entstehende Spannung $V_{R1}$ an den komplexen Leitwert des ersten Referenzleitwerts $Y_{R1}$ 112 angelegt, und wenn das Signal 102 an die zweite Übertragungsfunktion $A_R$ 108 angelegt wird, wird die entstehende Spannung $V_{R2}$ an den zweiten komplexen Leitwert 114 ($Y_{R2}$) angelegt. Jeder der entstehenden Ströme wird in einem Addierer 116 summiert. Wenn nur ein Pfad ausgewählt wird, wird nur eines dieser Signale nicht Null sein. Der sich ergebende Strom ist dann $I_C$, wenn die Zelle ausgewählt wird, $I_{R1}$ wenn die erste Referenz ausgewählt wird, und $I_{R2}$ wenn die zweite Zelle ausgewählt wird. Der entstehende Strom fließt durch einen Schaltkreis mit einer Übertragungsfunktion H 120, wobei H eine Übertragungsfunktion zur Umsetzung komplexen Stroms in eine komplexe Spannung für Messzwecke ist. Die entstehende, durch den Knoten 121 gemessene Spannung repräsentiert den komplexen Strom durch entweder den Leitwert der Zelle 13 oder ein der Referenzleitwerte. Die reellen (phasengleichen) und imaginären (Quadratur-) Komponenten dieser Spannung werden festgestellt, indem die Spannung an den Unterschaltkreis angelegt wird, der aus den Blöcken 122, 123 und 124 besteht, wie in Figur 5 gezeigt. Auf diese Weise werden daher Spannungen $U_{cm1}$ und $U_{cm2}$ gemessen, die den komplexen Strom durch die Zelle repräsentieren. Durch das Auswählen je einer der Referenzen können auch Spannungen gemessen werden, die den komplexen Strom durch die erste Referenz oder die zweite Referenz repräsentieren.

Zusätzlich zum Messen von Spannungen, die die komplexen Stromwerte repräsentieren, werden entsprechend des Schaltkreises auch Spannungen berechnet, die die komplexen Spannungswerte repräsentieren. Die Spannungen von der Zelle, $V_C$, der ersten Referenz, $V_{R1}$, und der zweiten Referenz, $V_{R2}$, werden an einen Addierer 118 angelegt. Da nur eine der Referenzen oder die Zelle gleichzeitig ausgewählt werden, wird nur einer dieser Werte von Null verschieden sein. Das Ergebnis wird an eine Übertragungsfunktion 126 angelegt, die eine komplexe Spannung am Knoten 127 ergibt. Die reellen und imaginären (phasengleichen und Quadratur-) Komponenten dieser Spannung werden durch Anlegen der Spannung an den Unterschaltkreis festgestellt, der aus den Blöcken 128, 129 und 130 besteht, wie in Figur 5 dargestellt.

Auf diese Weise ermöglicht der in Figur 5 dargestellte Schaltkreis eine Bestimmung der Real- und Imaginärteile sowohl der Spannung als auch des Stroms, der bzw. die mit einem ausgewählten Leitwert verbunden ist oder sind. Dieser Leitwert ist entweder mit der Zelle 13 des Kornfeuchtigkeitssensors 12 oder mit einer der Referenzleitwerte des Kornfeuchtigkeitssensors verbunden.

Zur weiteren Erklärung liegen die folgenden mathematischen Beziehungen vor:

$$G = W/V$$

$$H = U/I$$

In jedem Fall sind die jeweiligen Übertragungsfunktionen als Verhältnis des Ausgangswerts der Funktion zum Eingangswert der Funktion definiert. Außerdem ist der Leitwert mathematisch definiert als:

$$Y = I/V = (U/H)/(W/G) = (U/W) \cdot (G/H).$$

Sind diese allgemeinen Beziehungen gegeben, ist der Leitwert einer Referenz definiert als:

$$Y_R = U_R/W_R \cdot G/H.$$

Außerdem werden die Leitwerte der leeren Zelle 13, $Y_{CE}$, und einer vollen Zelle, $Y_{CF}$, folgendermaßen berechnet:

$$Y_{CE} = U_{CE}/W_{CE} \cdot G/H.$$

$$Y_{CF} = U_{CF}/W_{CF} \cdot G/H.$$

Wenn die Messungen für den Referenzleitwert und den Leitwert der Zelle bei denselben Umgebungsbedingungen durchgeführt werden, kann angenommen werden, dass sowohl H als auch G in den Leitwertgleichungen für die Zelle und die Referenzen gleich sind. Dann kennzeichnet das Folgende die Kalibrierfaktoren für die leere Zelle und die Referenzen:

$$W_R/U_R \cdot Y_R = W_{CE}/U_{CE} \cdot Y_{CE} => F = W_{CE}/U_{CE} \cdot U_R/W_R = Y_R/Y_{CE}$$

Der Kalibrierfaktor F der Referenz gibt das Verhältnis vom Leitwert der Referenz zum Leitwert der leeren Zelle bei denselben Umgebungsbedingungen wieder. Daher kann zu Kalibrierzwecken ein Referenzleitwert anstelle eines Leitwerts der leeren Zelle verwendet werden.

Unter der Annahme, dass F konstant bleiben wird, kann die komplexe Dielektrizitätskonstante der Kornprobe berechnet werden zu:

$$\varepsilon = (U_{CF} \cdot W_R)/(W_{CF} \cdot U_R) \cdot F,$$

wobei

$$\varepsilon = \varepsilon' - j\,\varepsilon''.$$

Daher ermöglicht die vorliegende Erfindung eine Messung des komplexen Leitwerts des Korns zu Feuchtigkeitsmesszwecken.

Um genaue Strom- und Spannungsmessungen durchzuführen, ist es zweckmäßig, dass die phasengleichen (IP) und Quadratur- (Q) Signale der lokalen Oszillatoren, die bei den Mischern 216, 220 und 224 zum Extrahieren der reellen und imaginären Komponenten komplexer Signale verwendet werden, eine Phasendifferenz von genau 90 Grad und an ihren Grundfrequenzen identische Amplituden haben. Fehler werden in dem Maß eingeführt, in dem das nicht der Fall ist. Durch die Verwendung zweier Referenzleitwerte mit bekannten und stabilen Werten werden jedoch Korrekturen dieser Fehler durchgeführt.

Die D-Funktionen 124 und 130 repräsentieren die Verzerrung des Imaginärteils gegenüber dem Realteil aller gemessener komplexer Werte. Alle gemessenen Werte $U_m$ und $W_m$ können durch Verwendung derselben Formel korrigiert werden, um $U$ und $W$ zu erhalten, welches die Werte sind, bevor jegliche Messwertverzerrfehler eingeführt werden.

Folgendes ist die verzerrte Beziehung zwischen den komplexen Spannungen, die die Ströme durch die Zelle und die Referenzen und ihre gemessenen Werte repräsentieren:

$$U = [1\ j] \cdot D^{-1} \cdot U_m,$$

wobei

$$D^{-1} = PFC = \begin{bmatrix} 1 & 0 \\ Pfc1 & pfc2 \end{bmatrix}$$

$$U_m = \begin{bmatrix} U_{m1} \\ U_{m2} \end{bmatrix}$$

Dieselbe verzerrte Beziehung gilt zwischen den komplexen Spannungen, die die Zell- und Referenzspannungen und ihre gemessenen Werte repräsentieren:

$$W = [1\ j] \cdot D^{-1} \cdot W_m,$$

wobei

$$D^{-1} = PFC = \begin{bmatrix} 1 & 0 \\ Pfc1 & pfc2 \end{bmatrix}$$

$$\overline{W}_m = \begin{matrix} \overline{W}_{m1} \\ \\ \overline{W}_{m2} \end{matrix}$$

Das Expandieren der obigen Gleichungen ergibt:

$$U = U_{m1} + j\cdot(pfc1\cdot U_{m1} + pfc2\cdot U_{m2})$$

$$W = W_{m1} + j\cdot(pfc1\cdot W_{m1} + pfc2\cdot W_{m2})$$

Die Korrekturfaktoren pfc1 und pfc2 werden durch die Verwendung zweier unterschiedlicher Referenzen herausgefunden, die bekannte und stabile Leitwerte mit verschiedenen Phasenwinkeln haben. Als Beispiel ist in einer Ausführungsform der Erfindung der erste Referenzwert ein temperaturstabiler Kondensator mit 1 % Toleranz (COG) mit einem Wert von 15 pF (Leitwert $Y_{R1}$) und die zweite Referenz ein Präzisionswiderstand mit 0,1 % Toleranz mit einem Wert von 2000 $\Omega$ (Leitwert $Y_{R2}$). Auch andere Referenzwerte können genutzt werden.

Das Verhältnis der Referenzleitwerte wird folgendermaßen berechnet:

$$R = Y_{R1}/Y_{R2} = Q_R + j\cdot Q_I.$$

Das Verhältnis der rohen Messwerte der zwei Referenzen ist:

$$R_m = (U_{R1}\cdot W_{R2})/(W_{R1}\cdot U_{R2}).$$

In die obige Gleichung können die Ergebnisse der Messungen eingefügt werden, so dass man erhält:

$$R_m = ((U_{R1m1}+j(U_{R1m1}\cdot pfc1+U_{R1m2}\cdot pfc2))(W_{R2m1}+j(W_{R2m1}\cdot pfc1+W_{R2m2}\cdot pfc2)))$$

$$/((W_{R1m1}+j(W_{R1m1}\cdot pfc1+W_{R1m2}\cdot pfc2))(U_{R2m1}+j(U_{R2m1}\cdot pfc1+U_{R2m2}\cdot pfc2))).$$

$R_m$ wird mit R gleichgesetzt und zwei quadratische Gleichungen mit zwei Unbekannten (pfc1, pfc2) werden abgeleitet:

$$a_1\cdot pfc1^2 + b_1\cdot pfc2^2 + c_1\cdot pcf1\cdot pfc2 + d_1\cdot pfc1 + e_1\cdot pfc2 + f_1 = 0$$

(vom Realteil)

$$a_2\cdot pfc1^2 + b_2\cdot pfc2^2 + c_2\cdot pcf1\cdot pfc2 + d_2\cdot pfc1 + e_2\cdot pfc2 + f_2 = 0$$

(vom Imaginärteil),
wobei
$a_1 = Q_R\ W_{R1m1}\cdot U_{R2m1} - U_{R1m1}\cdot W_{R2m1}$
$a_2 = Q_I\ W_{R1m1}\cdot U_{R2m1}$
$b_1 = Q_R\ W_{R1m2}\cdot U_{R2m2} - U_{R1m2}\cdot W_{R2m2}$
$b_2 = Q_I\ W_{R1m2}\cdot U_{R2m21}$
$C_1 = Q_R\ W_{R1m1}\cdot U_{R2m2} + Q_R \cdot W_{R1m2}\cdot U_{R2m1} - U_{R1m1}\cdot W_{R2m2} - U_{R1m2}\cdot W_{R2m1C}$
$c_2 = e_1$
$d_1 = 2 \cdot Q_I\ W_{R1m1}\cdot U_{R2m1}$
$d_2 = - 2 \cdot a_1$

$$e_1 = Q_I \cdot ( W_{R1m1} \cdot U_{R2m2} + W_{R1m2} \cdot U_{R2m1})$$
$$e_2 = - c_1$$
$$f_1 = - a_1$$
$$f_2 = - a_2$$

Diese zwei quadratischen Gleichungen werden dann gleichzeitig für pfc1 und pfc2 gelöst. Da eine einfache, geschlossene Form nicht vorhanden ist, können sie beispielsweise durch Newton-Raphson-Iteration gelöst werden. Andere Algorithmen zur Lösung numerischer Gleichungen können ebenfalls verwendet werden. Es ist bekannt, dass die Lösung nahe des Punktes (pcf1=0, pfc2=1) liegt, so dass diese vorzugsweise als Startpunkt verwendet werden. Theoretisch sind vier verschiedene Lösungen möglich. Jede Lösung, die nicht nahe (0,1) liegt, sollte als ungeeignet angesehen werden. Bei einer Softwareimplementation kann eine geeignete Fehlerbedingung gesetzt werden. Es ist nicht wahrscheinlich, dass dies vorkommt, jedoch können Vorsichtsmaßnahmen für den Fall, dass es vorkommt, vorgesehen werden.

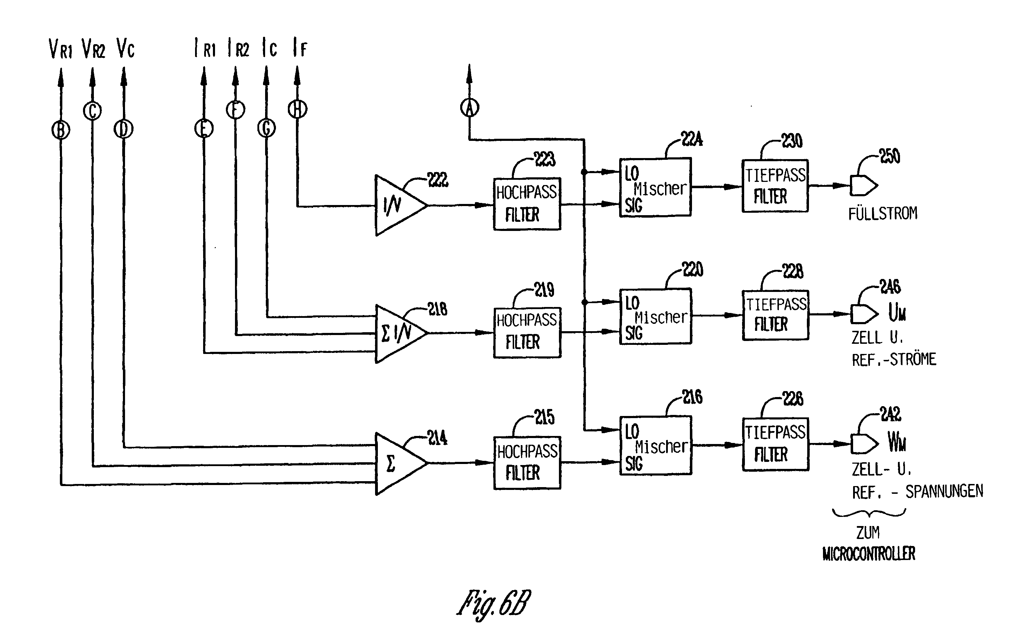

Die Figuren 6A und 6B zeigen ein Schema für den erfindungsgemäßen Kornfeuchtigkeitssensor. Das Schema zeigt eine Anzahl an Eingangs- und Ausgangsleitungen zur Verbindung mit einer intelligenten Steuerung, wie einem Prozessor, Mikrocontroller, integriertem Schaltkreis oder anderem Gerät. Dieses Schema zeigt nur eine Schaltkreiskonfiguration gemäß der vorliegenden Erfindung. Die vorliegende Erfindung stellt die Möglichkeit bereit, selektiv eine von einer Mehrzahl komplexer Leitwerte bei einer Vielzahl von Frequenzen zu messen.

Die Eingänge in das System (Ausgänge einer intelligenten Steuerung) werden in Figur 6A gezeigt. Die Eingänge umfassen einen ersten Frequenzeingang 164 und einen zweiten Frequenzeingang 166. Optional werden ein erster Sinuswellengenerator 178 und ein zweiter Sinuswellengenerator 180 verwendet. Die Sinuswellengeneratoren nehmen den Rechteckwellenausgang eines Mikrocontrollers, teilen die Frequenz wie erforderlich und glätten den Ausgang, so dass ein sinusförmiges Signal erzeugt wird. Der Ausgang des ersten Sinuswellengenerators 178 ist elektrisch mit drei Schaltereingängen eines doppelten Umschalters 198 mit vier Eingängen verbunden. Zusätzlich ist der Ausgang des ersten Sinuswellengenerators 178 elektrisch mit einem 90° Phasenschieber 194 verbunden. Der 90° Phasenschieber 194 ist derart aufgebaut, dass sein Ausgangssignal um 90° in der Phase gegenüber seinem Eingangssignal verschoben ist. Der 90° Phasenschieber ist elektrisch mit dem Schaltereingang des doppelten Umschalters 198 verbunden. Der Ausgang des zweiten Sinuswellengenerators 180 ist ähnlich angeschlossen.

Der erste Sinuswellengenerator 178 und der zweite Sinuswellengenerator 180 arbeiten bei unterschiedlichen Frequenzen. Beispielsweise arbeitet der erste Sinuswellengenerator 178 bei 10 MHz, während der zweite Sinuswellengenerator bei 1 MHz arbeitet.

Der doppelte Umschalter 198 wird durch die Eingänge 172 und 174 gesteuert, die verwendet werden, eines der Signale auszuwählen. Einer der Ausgänge des Schalters 198 ist elektrisch mit einem Eingang des doppelten Umschalters 200 mit zwei Ausgängen verbunden. Eingänge 168 und 170 sind mit dem Umschalter 200 verbunden, um zu steuern, welcher der Ausgänge ausgewählt wird. Die Ausgänge sind gepuffert und dann elektrisch mit der Sensorzelle 208 (die der Zelle 13 in den anderen Figuren entspricht), einem ersten Referenzleitwert 210 und einem zweiten Referenzleitwert 212 verbunden. Die Referenzleitwerte werden für Kalibrierzwecke verwendet.

Wie in Figur 6B gezeigt, sind die gepufferten Ausgänge, die die Zelle 208 und die beiden Referenzen treiben, auch elektrisch mit einem Summierkreis 214 verbunden. Der Ausgang des Summierkreises ist elektrisch über einen Hochpassfilter 215 mit einem Mischer 216 verbunden. Der Mischer 216 hat auch einen lokalen Oszillatoreingang, der elektrisch mit einem Ausgang des Schalters 198 (Figur 6A) verbunden ist. Der Ausgang des Mischers 216 geht durch einen Tiefpassfilter 226 und ist dann elektrisch mit einem Analog/Digitalwandler verbunden und wird durch den Mikrocontroller ausgelesen. Am Ausgang des Mischers 216 liegt eine Gleichspannung an, die proportional zur Komponente des Eingangsspannungssignals ist, welche mit dem lokalen Oszillator gleichphasig ist.

Die Sensorplatte 68 der Zelle 208 und die erste Referenz 210 und die zweite Referenz 212 aus Figur 6A sind elektrisch mit einem summierenden Strom/Spannungsumsetzer 218 verbunden, wie er in Figur 6B gezeigt ist. Der summierende Strom/Spannungsumsetzer 218 hat einen Eingang mit niedriger Impedanz, der virtuell auf Erdpotential liegt. Der Ausgang des summierenden Strom/Spannungsumsetzers 218 ist über ein Hochpassfilter 219 elektrisch mit einem zweiten Mischer 220 verbunden. Der zweite Mischer 220 hat auch einen lokalen Oszillatoreingang, der elektrisch mit einem Ausgang des Schalters 198 (Figur 6A) verbunden ist. Der Ausgang des Mischers 220 geht durch einen Tiefpassfilter 228 und ist dann elektrisch mit einem Analog/Digitalwandler verbunden und wird durch den Mikrocontroller ausgelesen. Am Ausgang des Mischers 220 liegt eine Gleichspannung an, die proportional zur Komponente des Eingangssignals ist, welches mit dem lokalen Oszillator gleichphasig ist.

Außerdem läuft der Strom $I_F$ von der Füllstandssensorplatte 66 an der Zelle 208 (s. Figur 6A) durch den Strom/Spannungsumsetzer 222. Dieser Strom/Spannungsumsetzer 222 hat ebenfalls einen Eingang mit niedriger Impedanz, der sich virtuell auf Erdpotential befindet. Der Ausgang des Strom/Spannungsumsetzers 222 ist über ein Hochpassfilter 223 mit einem dritten Mischer 224 verbunden. Der dritte Mischer 224 hat auch einen lokalen Oszillatoreingang, der elektrisch mit einem Ausgang des Schalters 198 verbunden ist. Der Ausgang des Mischers 224 geht über einen Tiefpassfilter 230 an einen Analog/Digitalwandler und wird vom Mikrocontroller ausgelesen. Diese Konfiguration ermöglicht eine Überwachung des Leitwerts der Füllstandssensorplatte 66 gegenüber der Sensorplatte 68. Wenn dieses Verhältnis proportional zu den relativen Größen der Platten 66, 68 ist, dann wird die Zelle 208 als mit Korn gefüllt angesehen.

Das synchrone Nachweisverfahren der Messung komplexer Signale unter Verwendung eines lokalen Oszillators, eines Mischers und eines Tiefpassfilters, wie es oben beschrieben wurde, hat einen sehr engen Bandpassfiltereffekt, der den Untergrundgeräuscheinfluss auf die Messung wesentlich reduziert. Das Verfahren der Messung sehr kleiner Ströme mit virtueller Erde wird genutzt, um den Vorteil einer wesentlichen Verminderung des Einflusses parasitärer Elemente auf den Stromsummier- und Messknoten zu erzielen.

In der Figur 6A ist ein Thermistor oder ein anderer Temperatursensor an der elektrisch beaufschlagten Platte 64 der Zelle 208 angebracht. Dies ist nur ein Beispiel der Anbringung eines Temperatursensors. Der Temperatursensor kann auch an einer der anderen Platten der Zelle 208 angebracht sein. Die Messung der Temperatur erlaubt eine entsprechende Korrektur der Feuchtigkeitsberechnungen.

## Patentansprüche

1. Kornfeuchtigkeitssensor für einen Mähdrescher, mit einer elektrisch beaufschlagten Platte (64), einer Sensorplatte (68), die in der Nähe der elektrisch beaufschlagten Platte (64) angeordnet und zu ihr im Wesentlichen parallel orientiert ist, um im Zwischenraum zwischen den Platten (64, 68) die Kapazität zu messen, einer Füllstandssensorplatte (66), die in der Nähe der Sensorplatte (68) und im Wesentlichen parallel zur elektrisch beaufschlagten Platte (64) angeordnet ist, um zu erfassen, ob der Zwischenraum mit Korn gefüllt ist und mit einer Steuerung des Kornfeuchtigkeitssensors, **dadurch gekennzeichnet, dass** die Steuerung ausgelegt ist, das Verhältnis des Leitwerts der Füllstandssensorplatte (66) zum Leitwert der Sensorplatte (68) mit dem Verhältnis der Größen der Füllstandssensorplatte (66) und der Sensorplatte (68) zu vergleichen und den Kornfeuchtigkeitssensor als gefüllt anzusehen, wenn das Verhältnis der Leitwerte dem Größenverhältnis entspricht.

2. Kornfeuchtigkeitssensor nach Anspruch 1, **gekennzeichnet durch** eine Abschirmung (70), die im Abstand von der Sensorplatte (68) und parallel dazu auf der von der elektrisch beaufschlagten Platte (64) abgewandten Seite der Sensorplatte (68) angeordnet ist.

3. Kornfeuchtigkeitssensor nach Anspruch 2, **dadurch gekennzeichnet, dass** die Abschirmung sich auf demselben elektrischen Potential wie die Sensorplatte (68) befindet.

4. Kornfeuchtigkeitssensor nach einem der Ansprüche 1 bis 3, **dadurch gekennzeichnet, dass** ein Temperatursensor mit der die Platten (64, 66, 68) enthaltenden Zelle (13, 208) gekoppelt ist, um eine etwa der Korntemperatur entsprechende Temperatur zu erfassen.

5. Kornfeuchtigkeitssensor nach einem der Ansprüche 1 bis 4, **dadurch gekennzeichnet, dass** eine Mehrzahl auswählbarer Signaleingänge mit der elektrisch beaufschlagten Platte (64) koppelbar sind, die jeweils mit einer zugeordneten Frequenz arbeiten.

6. Kornfeuchtigkeitssensor nach Anspruch 5, **dadurch gekennzeichnet, dass** eine Mehrzahl von Referenzleitwerten mit der Mehrzahl auswählbarer Signaleingänge verbindbar sind, um den Kornfeuchtigkeitssensor zu kalibrieren.

7. Kornfeuchtigkeitssensor nach einem der Ansprüche 1 bis 6, **dadurch gekennzeichnet, dass** die elektrisch beaufschlagte Platte (64) mit unterschiedlichen Frequenzen beaufschlagbar ist, dass der komplexe Leitwert zwischen der elektrisch beaufschlagten Platte (64) und der Sensorplatte (68) mit dazwischen eingefülltem Korn messbar ist, dass eine Referenz (210, 212) mit demselben Signal beaufschlagbar ist und ihr komplexer Leitwert messbar ist, und dass eine komplexe Dielektrizitätskonstante aus den beiden gemessenen Leitwerten errechenbar ist.

8. Kornfeuchtigkeitssensor nach Anspruch 7, **dadurch gekennzeichnet, dass** beim Errechnen der komplexen Dielektrizitätskonstante ein Kalibrierfaktor für die Referenz (210, 212) errechenbar ist, um den Leitwert der leeren Zelle (13, 208) zu berechnen.

9. Kornfeuchtigkeitssensor nach Anspruch 7 oder 8, **dadurch gekennzeichnet, dass** unter mehreren Referenzen (210, 212) auswählbar ist.

10. Kornfeuchtigkeitssensor nach einem der Ansprüche 1 bis 9, **dadurch gekennzeichnet, dass** die Real- und Imaginärkomponenten der an die elektrisch beaufschlagte Platte (64) angelegten Spannung messbar sind, dass die Real- und Imaginärkomponenten des an der Sensorplatte (68) fließenden Stroms messbar sind, und dass aus der Spannung und dem Strom die komplexe Dielektrizitätskonstante des Korns errechenbar ist.

11. Kornfeuchtigkeitssensor nach Anspruch 10, **dadurch gekennzeichnet, dass** die Spannung und/oder der Strom durch einen Synchrondetektor, insbesondere Mischer (216, 220), messbar ist.

12. Kornfeuchtigkeitssensor nach Anspruch 11, **dadurch gekennzeichnet, dass** der Synchrondetektor zwischen einer Messung des Realteils und des Imaginärteils der Spannung bzw. des Stroms umschaltbar ist.

**Claims**

1. A grain moisture sensor for a combine harvester, with an electrified plate (64), a sensor plate (68) which is disposed in the vicinity of the electrified plate (64) and is orientated substantially parallel thereto, in order to measure the capacitance in the space between the plates (64, 68), a state of fill sensor plate (66) which is arranged in the vicinity of the sensor plate (68) and substantially parallel to the electrified plate (64), in order to detect whether the space is filled with grain, and with a controller of the grain moisture sensor, **characterized in that** the controller is arranged to compare the ratio of the conductance of the state of fill sensor plate (66) to the conductance of the sensor plate (68) with the ratio of the sizes of the state of fill sensor plate (66) and the sensor plate (68) and to treat the grain moisture sensor as filled when the ratio of the conductances corresponds to the ratio of sizes.

2. A grain moisture sensor according to claim 1, **characterized by** screen (70) which is arranged spaced from the sensor plate (68) and parallel thereto on the side of the sensor plate (68) facing away from the electrified plate (64).

3. A grain moisture sensor according to claim 2, **characterized in that** the screen is at the same electric potential as the sensor plate (68).

4. A grain moisture sensor according to any of claims 1 to 3, **characterized in that** a temperature sensor is coupled to the cell (13, 208) containing the plates (64, 66, 68), in order to detect a temperature corresponding approximately to the grain temperature.

5. A grain moisture sensor according to any of claims 1 to 4, **characterized in that** a plurality of selectable signal inputs can be coupled to the electrified plate (64), each operating with an associated frequency.

6. A grain moisture sensor according to claim 5, **characterized in that** a plurality of reference conductances can be connected to the plurality of signal inputs, in order to calibrate the grain moisture sensor.

7. A grain moisture sensor according to any of claims 1 to 6, **characterized in that** the electrified plate (64) can have different frequencies applied thereto, **in that** a complex conductance between the electrified plate (64) and the sensor plate (68) can be measured with grain filled in between them, **in that** a reference (210, 212) can have the same signal applied thereto and its complex conductance measured, and **in that** a complex dielectric constant can be computed from the two measured conductances.

8. A grain moisture sensor according to claim 7, **characterized in that**, on computing the complex dielectric constant a calibration factor for the reference (210, 212) can be computed, in order to compute the conductance of the empty cell (13, 208).

9. A grain moisture sensor according to claim 7 or 8, **characterized in that** a selection can be made between a plurality of references (210, 212).

10. A grain moisture sensor according to any of claims 1 to 9, **characterized in that** the real and imaginary components of the voltage applied to the electrified plate (64) can be measured, **in that** the real an imaginary components of the current flowing at the sensor plate (68) can be measured, and **in that** the complex dielectric constant of the grain can be computed from the voltage and the current.

11. A grain moisture sensor according to claim 10, **characterized in that** the voltage and/or the current can be measured by a synchronous detector, especially a mixer (216, 220).

**12.** A grain moisture sensor according to claim 11, **characterized in that** the synchronous detector can be switched between a measurement of the real part and of the imaginary part of the voltage or the current.

**Revendications**

**1.** Capteur d'humidité des céréales pour une moissonneuse-batteuse, comportant une plaque (64) sollicitée électriquement, une plaque de détection (68), qui est agencée à proximité de la plaque (64) sollicitée électriquement et est orientée sensiblement parallèlement à celle-ci, en vue de mesurer la capacité dans l'espace intermédiaire entre les plaques (64, 68), une plaque de détection du niveau de remplissage (66), qui est agencée à proximité de la plaque de détection (68) et est orientée sensiblement parallèlement à la plaque (64) sollicitée électriquement, en vue de détecter si l'espace intermédiaire est rempli de céréales, et avec une commande du capteur d'humidité des céréales, **caractérisé en ce que** la commande est conçue pour comparer le rapport entre la conductance de la plaque de détection du niveau de remplissage (66) et la conductance de la plaque de détection (68) avec le rapport entre les grandeurs de la plaque de détection du niveau de remplissage (66) et de la plaque de détection (68) et pour considérer que le capteur d'humidité des céréales est rempli lorsque le rapport des conductances correspond au rapport des grandeurs.

**2.** Capteur d'humidité des céréales selon la revendication 1, **caractérisé par** un écran (70), qui est agencé à distance de la plaque de détection (68) et parallèlement à celle-ci sur le côté de la plaque de détection (68) détourné de la plaque (64) sollicitée électriquement.

**3.** Capteur d'humidité des céréales selon la revendication 2, **caractérisé en ce que** l'écran (70) est situé sur le même potentiel électrique que la plaque de détection (68).

**4.** Capteur d'humidité des céréales selon l'une quelconque des revendications 1 à 3, **caractérisé en ce qu'**un capteur de température est couplé à la cellule (13, 208) contenant les plaques (64, 66, 68) en vue de détecter une température correspondant pratiquement à la température des céréales.

**5.** Capteur d'humidité des céréales selon l'une quelconque des revendications 1 à 4, **caractérisé en ce qu'**une pluralité de sorties de signaux sélectionnables peuvent être couplées à la plaque (64) sollicitée électriquement, lesquelles fonctionnent toutes avec une fréquence correspondante.

**6.** Capteur d'humidité des céréales selon la revendication 5, **caractérisé en ce qu'**une pluralité de conductances de référence peuvent être reliées à la pluralité de sorties de signaux sélectionnables, en vue d'étalonner le capteur d'humidité des céréales.

**7.** Capteur d'humidité des céréales selon l'une quelconque des revendications 1 à 6, **caractérisé en ce que** la plaque (64) sollicitée électriquement peut être sollicitée par différentes fréquences, **en ce que** la conductance complexe entre la plaque (64) sollicitée électriquement et la plaque de détection (68) est mesurable avec les céréales contenues entre les deux plaques, **en ce qu'**une référence (210, 212) peut être sollicitée avec le même signal et sa conductance complexe est mesurable, et **en ce qu'**une constante diélectrique complexe peut être calculée à partir des deux conductances mesurées.

**8.** Capteur d'humidité des céréales selon la revendication 7, **caractérisé en ce que** pendant le calcul de la constante diélectrique complexe, il est possible de calculer un facteur d'étalonnage pour la référence (210, 212) en vue de calculer la conductance de la cellule vide (13, 208).

**9.** Capteur d'humidité des céréales selon la revendication 7 ou 8, **caractérisé en ce qu'**il est possible de sélectionner parmi plusieurs références (210, 212).

**10.** Capteur d'humidité des céréales selon l'une quelconque des revendications 1 à 9, **caractérisé en ce qu'**il est possible de mesurer les composantes réelles et les composantes imaginaires de la tension appliquée à la plaque (64) sollicitée électriquement, **en ce qu'**il est possible de mesurer les composantes réelles et les composantes imaginaires du courant circulant au niveau de la plaque de détection (68) et **en ce qu'**il est possible de calculer à partir de la tension et du courant la constante diélectrique complexe des céréales.

**11.** Capteur d'humidité des céréales selon la revendication 10, **caractérisé en ce que** la tension et/ou le courant peuvent être mesurés par un détecteur synchrone, en particulier un mélangeur (216, 220).

12. Capteur d'humidité des céréales selon la revendication 11, **caractérisé en ce que** le détecteur synchrone est commutable entre une mesure de la part réelle et de la part imaginaire de la tension ou du courant.

EP 1 305 994 B1

*Fig.1A*

*Fig. 1B*

*Fig. 1C*

Fig.2

Fig.3

Fig. 4

Fig. 5

*Fig. 6A*

Fig. 6B

$V_{R1}$  $V_{R2}$  $V_C$    $I_{R1}$  $I_{R2}$  $I_C$  $I_F$

Ⓑ  Ⓒ  Ⓓ    Ⓔ  Ⓕ  Ⓖ  Ⓗ    Ⓐ

222 I/N
223 HOCHPASS FILTER
22A LO Mischer SIG
230 TIEFPASS FILTER
250 FÜLLSTROM

218 ΣI/N
219 HOCHPASS FILTER
220 LO Mischer SIG
228 TIEFPASS FILTER
246 $U_M$
ZELL U. REF.-STRÖME

214 Σ
215 HOCHPASS FILTER
216 LO Mischer SIG
228 TIEFPASS FILTER
242 $W_M$
ZELL- U. REF. - SPANNUNGEN

ZUM MICROCONTROLLER

EP 1 305 994 B1